Europäisches Patentamt

European Patent Office

Office européen des brevets

(19)

(11) EP 0 920 813 A1

(12) DEMANDE DE BREVET EUROPEEN

(43) Date de publication:
09.06.1999 Bulletin 1999/23

(51) Int. Cl.⁶: **A23L 1/305**, A23L 1/05

(21) Numéro de dépôt: 97203840.0

(22) Date de dépôt: 08.12.1997

(84) Etats contractants désignés:
AT BE CH DE DK ES FI FR GB GR IE IT LI LU MC NL PT SE
Etats d'extension désignés:
AL LT LV MK RO SI

(71) Demandeur:
SOCIETE DES PRODUITS NESTLE S.A.
1800 Vevey (CH)

(72) Inventeurs:
• Dangin, Martial
63000 Clermont-Ferrand (FR)
• Garcia-Rodenas, Clara Lucia
1074 Mollie-Margot (CH)
• Beaufrere, Bernard
63400 Chamalieres (FR)
• Ballevre, Olivier
1012 Lausanne (CH)

(54) **Accélération de la vitesse de digéstion d'une protéine**

(57) L'invention concerne ainsi un procédé pour accélérer la vitesse de digestion d'une matière protéique, dans lequel on traite une matière protéique avec la transglutaminase, et on la mélange avec des polysaccharides anioniques. L'invention concerne aussi l'utilisation d'une matière protéique à digestion rapide pour la préparation d'une composition alimentaire ou pharmaceutique destinée à être administrée oralement à un mammifère afin d'induire un pic postprandial d'augmentation plasmatique en acides aminés. Cette composition est destinée à pouvoir moduler le gain protéique postprandial, et/ou de limiter les problèmes liés aux troubles de la motilité gastro-intestinale, et/ou de limiter les sensations postprandiales de nausée chez les femmes enceintes, et/ou de limiter les risques postprandiaux de régurgitation et/ou de reflux gastro-oesophagien. Enfin, l'invention a aussi pour objet une composition alimentaire ou pharmaceutique comprenant des polysaccharides anioniques et une matière protéique traitée par la transglutaminase.

FIGURE 3

EP 0 920 813 A1

Printed by Xerox (UK) Business Services
2.16.7/3.6

## Description

L'invention a pour objet une méthode pour accélérer la vitesse de digestion d'une protéine, et l'utilisation d'une protéine ainsi modifiée pour la préparation d'une composition alimentaire ou pharmaceutique permettant de moduler le taux plasmatique postprandial en acides aminés.

Etat de la technique

Chez l'homme, au cours d'un nycthémère, la prise alimentaire est discontinue. Des périodes postprandiales, c'est à dire les phases d'assimilation des nutriments à partir du tube digestif, alternent avec des périodes de jeûne physiologique. Ces variations diurnes du statut nutritionnel affectent les composantes du métabolisme protéique et par conséquent le bilan protéique.

Ainsi, la consommation de protéines résulte en une augmentation du taux plasmatique en acides aminé (Aoki *et al.*, Am. J. Clin. Nutr., <u>41</u>, 1-18, 1987). De même, l'élévation du taux plasmatique en acides aminés est associée avec un déclin de la protéolyse et une stimulation de l'oxydation des acides aminés et de la synthèse protéique (Castellino *et al.,* Am. J. Physiol., <u>262</u>, 162-176, 1992; Giordano *et al.*, Diabetes, <u>45</u>, 393-399, 1996; Clugston *et al.*, Clin. Nutr., <u>36</u>, 57-70, 1982; Motil *et al.*, Am. J. Physiol., <u>240</u>, E712-721, 1981; Melville *et al.*, Metabolism., <u>30</u>, 248-255, 1989; Pacy *et al.*, Clin. Sci., <u>86</u>, 103-118, 1994).

Le bilan protéique négatif pendant la période de jeûne physiologique devient positif pendant la période postprandiale. L'importance relative de chaque phase détermine alors l'évolution de la masse protéique corporelle. Il est donc essentiel de pouvoir magnifier le gain protéique postprandial afin d'optimiser l'évolution de la masse protéique dans de nombreuses circonstances.

Récemment, Boirie *et al.* ont montré, chez des jeunes volontaires sains, que le gain protéique postprandial dépend de la vitesse de digestion des protéines ingérées (délai entre l'ingestion et l'absorption des nutriments par l'organisme), ce phénomène étant lié à la structures des protéines. Le lactosérum administré en charge orale unique induit une apparition très précoce dans le sang des acides aminés d'origine alimentaire, ce qui se traduit par un pic de concentration plasmatique en acides aminés. On observe aussi une nette stimulation de la synthèse protéique et de l'oxydation de la leucine. Ces modifications postprandiales conduisent à une importante amélioration du gain protéique par rapport à la période de jeûne physiologique (Boirie *et al.*, Am. J. Physiol., <u>271</u>, E1083-1091, 1996).

En comparaison avec le lactosérum, les acides aminés issus de la caséine sont absorbés progressivement sur une plus longue période. Dans ce cas, la concentration plasmatique en acides aminés s'élève modérément mais cette élévation se prolonge au cours du temps, plusieurs heures après un repas (Boirie *et al.*, Nutr. Clin. Metabol., <u>9,</u> 171, 1995).

Entre ces deux types de protéines, la vitesse de digestion est donc différente, le lactosérum étant ainsi classé parmi les protéines à digestion rapide, et la caséine étant classée parmi les protéines à digestion lente.

Le comportement des protéines à digestion rapide peut être mis à profit pour la nutrition de sujets ayant, pour assurer le maintien de leur masse corporelle, un besoin physiologique important et immédiat en acides aminés, comme les patients sortant d'une opération chirurgicale (Arnold *et al.*, Nutr. Clin. Metabol., <u>6</u>, 3-12, 1992); les enfants (Dewey *et al.*, Eur. J. Clin. Nutr., <u>50</u>, supplement 1, S119-150, 1996); ou les sportifs, en particulier après un effort physique, par exemple (Biolo *et al.*, Am. J. Physiol., 273, E122-129, 1997; Rennie *et al.*, In Physical Activity, Fitness and Health: Physical Activity & Protein Metabolism, Bouchard *et al.,* Champaign, IL, Human kinetics, 432-450, 1994).

Les protéines à digestion rapide peuvent être aussi mises à profit pour la nutrition de sujets ayant des nausées, des vomissements et/ou une sensation de satiété trop élevée et prolongée après un repas de sorte qu'ils ont une gêne, voire des difficultés, à se nourrir régulièrement et/ou suffisamment. Les personnes concernées peuvent être sujettes à des nausées passagères comme les femmes enceintes; ou peuvent présenter des troubles de la motilité gastro-intestinale notamment les personnes âgées et les personnes présentant un état pathologique comme une anorexie mentale, un diabète, la présence d'un carcinome gastrique, des désordres neurologiques (la maladie de Parkinson), une dépendance aux drogues ou à l'alcool, etc., par exemple (Maes *et al.*, In $^{13}CO_2$-Breath test at the laboratory: Digestion-Absorption, Y. Groos Ed., p.55-69, 1996)

Les protéines à digestion rapide peuvent être aussi mises à profit pour limiter les risques de régurgitation et/ou de reflux gastro-oesophagien, notamment chez les nourrissons, les prématurés, les femmes enceintes ou les patients nourrit par voie entérale (Fried *et al.*, J. Pediatr., <u>120</u>, 569-572, 1992).

A ce jour, il n'a jamais été proposé d'accélérer la vitesse de digestion d'une protéine, notamment afin de moduler le taux plasmatique postprandial en acides aminés, et en conséquence afin de moduler le gain protéique postprandial, et/ou de limiter les sensations postprandiales de nausée chez la femme enceinte, et/ou de limiter les problèmes liés aux troubles de la motilité gastro-intestinale , et/ou de limiter les risques postprandiaux de régurgitation et de reflux gastro-oesophagien.

En outre, certaines protéines à digestion lente, comme la caséine, peuvent présenter une valeur nutritive élevée, c'est à dire une teneur équilibrée et élevée en chacun des acides aminés essentiels pour le corps, comme la lysine, le tryptophane, la leucine, l'isoleucine, la valine, la phénylalanine, la méthionine et la thréonine, par exemple. Malheureusement, la valeur nutritive de ces protéines n'est pas suffisamment mise à profit chez les personnes ayant un besoin physiologique en protéines à digestion rapide.

Certains traitements des protéines sont déjà connus pour modifier leur vitesse de digestion.

Par exemple, WO97/05785 (Milupa GmBH) indique que le traitement des protéines avec la transglutaminase, notamment la caséine du lait, permet de ralentir leur vitesse de digestion. La transglutaminase [EC2.3.2.13], appelée encore Factor XIII$_a$, $\gamma$-glutamyltransférase ou fibrinoligase, est connue pour modifier les protéines en catalysant des réactions de polymérisation, de désamidations et d'incorporation d'amines (Nielsen, Food biotechnol., 9, 119-156, 1995).

De même, les fibres diététiques solubles sont connues pour augmenter la viscosité du contenu gastro-intestinal, et donc le temps d'hydrolyse et d'absorption des sucres (Cameron-Smith et al., Br. J. Nutr., 71, 563-571, 1994). Dans le même sens, US5126332 (Terumo Kabushiki Kaisha) préconise de mélanger la caséine avec des caraghénanes ou une gomme de guar de sorte à former un gel dans l'estomac, qui sera lentement désagrégé dans l'intestin afin de ralentir l'absorption des sucres, et probablement aussi de la caséine, et ainsi de prévenir une augmentation rapide du glucose dans le sang de diabétiques.

Enfin, un autre moyen efficace pour accélérer la vitesse de digestion des protéines consiste généralement à procéder à une hydrolyse poussée, jusqu'à obtenir des résidus d'hydrolyse inférieurs à 10000 Dalton, par exemple. A cet effet, US5039532 décrit un procédé dans lequel, on soumet du petit-lait à une hydrolyse enzymatique partielle au moyen d'enzymes protéolytiques, on le soumet à un traitement thermique pour dénaturer les protéines mineures, les rendant ainsi accessibles à une dégradation enzymatique suivante, on le refroidit, puis on le soumet à une deuxième hydrolyse et à nouveau traitement thermique pour inactiver l'enzyme (voir aussi EP96202475.8; EP629350; JP 3-18168; Beaufrère et al., Am. J. Physiology, 30, E907-E914, 1994).

La présente invention vise à assurer les besoins nutritifs de certaines catégories de personnes, au moyen de protéines, initialement à digestion lente, qui ont été transformées en protéines à digestion rapide.

Résumé de l'invention

L'invention concerne ainsi un procédé pour accélérer la vitesse de digestion d'une matière protéique dans lequel, on traite une matière protéique avec la transglutaminase, et on la mélange avec des polysaccharides anioniques.

L'invention concerne aussi l'utilisation d'une matière protéique à digestion rapide, c'est à dire une matière qui, lorsqu'elle est ingérée par des rats de 140-200g, conduit à une disparition de la moitié de l'azote ingérée présente dans le tube digestif en moins de 70 min,

    pour la préparation d'une composition alimentaire ou pharmaceutique destinée à être administrée oralement à un mammifere afin d'induire un pic postprandial plasmatique en acides aminés, et en conséquence afin de moduler le gain protéique postprandial, et/ou de limiter les problèmes liés aux troubles de la motilité gastro-intestinale, et/ou de limiter les sensations postprandiales de nausée chez les femmes enceintes, et/ou de limiter les risques postprandiaux de régurgitation et/ou de reflux gastro-oesophagien,

    ladite matière protéique ayant été préalablement traitée de sorte à transformer les protéines à digestion lente qu'elle contenait en protéines à digestion rapide ayant conservé substantiellement au moins le même poids moléculaire initial.

Enfin, l'invention a aussi pour objet une composition alimentaire ou pharmaceutique comprenant des polysaccharides anioniques et une matière protéique traitée par la transglutaminase.

Description détaillée de l'invention

Contrairement à toutes attentes, on peut accélérer la vitesse de digestion d'une protéine sans procéder à son hydrolyse préalable.

De même, en dépit des convictions passées, les protéines traitées par la transglutaminase , et/ou les protéines auxquelles on adjoint des fibres anioniques présentent une vitesse de digestion accélérée. WO97/05785 (Milupa GmBH) prétendait, sans le démontrer, que le traitement des protéines avec la transglutaminase, notamment la caséine du lait, permettait de ralentir leur vitesse de digestion. De même, comme les fibres diététiques augmentent la viscosité du contenu gastro-intestinal, on estimait que le temps de digestion de tous les nutriments présents dans un repas contenant ces fibres devait être alors augmenté (voir à cet effet US5126332).

Enfin, les traitements selon l'invention visant à accélérer la vitesse de digestion des protéines ne se font pas au détriment de la digestibilité des protéines. Les protéines ainsi traitées n'induisent donc pas substantiellement une augmentation du rapport calculé entre la quantité de protéines ingérées oralement et la quantité de protéines absorbées par le corps (A titre d'exemple, voir Vaughan *et al.*, Am. J. Clin. Nutrition, 30, 1709-1712, 1977).

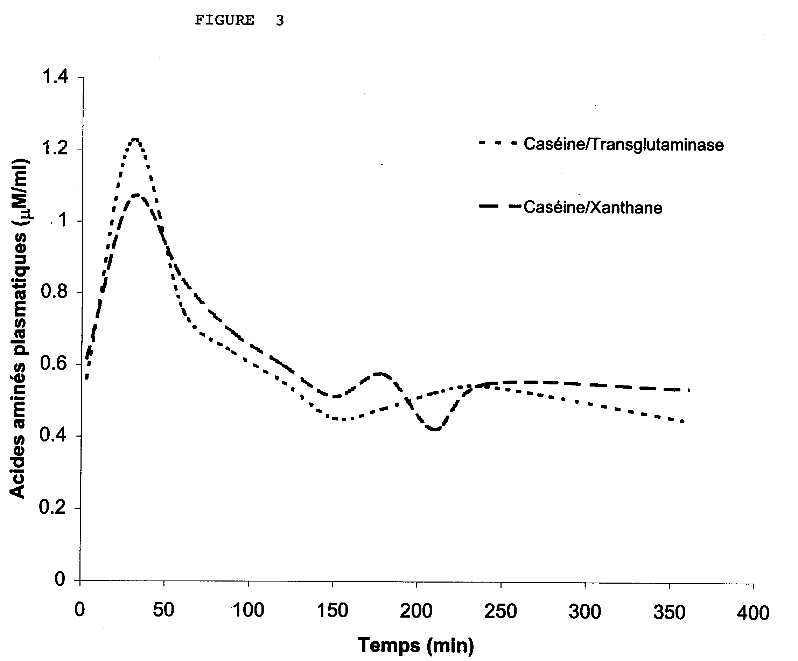

Dans le cadre de la présente invention, l'expression "pic postprandial plasmatique en acides aminés" correspond à une augmentation rapide et sensible du taux plasmatique en acides aminés après un repas, suivi d'une décroissance quasiment aussi rapide (voir la figure 3 ci-après).

Pour mettre en oeuvre le présent procédé, on utilise une matière protéique, c'est à dire tous genres de matières comprenant des protéines, qu'elles soient d'origine animale, végétale ou microbienne, notamment des protéines de lait, d'oléagineux, de légumineuses, de jaune d'oeuf ou de levures de bière, par exemple.

Par lait, on entend désigner, d'une part, un lait d'origine animale, tel que les laits de vache, de chèvre, de brebis, de bufflesse, de zébu, de jument, d'ânesse, de chamelle, etc. Le terme lait s'applique également à ce que l'on appelle communément un lait végétal, c'est à dire un extrait de matières végétales traités ou non, telles que les légumineuses (soja, pois chiche, lentille, etc...) ou des oléagineuses (colza, soja, sésame, coton, etc...), ledit extrait contenant des protéines en solution ou en suspension colloïdale, coagulables en milieu acide. Enfin, le mot lait désigne aussi des mélanges de laits animaux et de laits végétaux.

Les matières protéiques ayant une valeur nutritive élevée, selon les rations recommandées, sont particulièrement indiquées dans le cadre de la présente invention, comme la caséine et les protéines d'oeuf, du soja, du pois, du haricot, des lentilles, du pois chiche, du lupin, du caroube, du colza ou provenant d'autres sources reconnues pour leurs valeurs nutritives (FAO/WHO, Protein Quality Evaluation, n°51, Rome 1991). Ces protéines peuvent contenir une teneur équilibrée et élevée en chacun des acides aminés essentiels pour le corps, comme la lysine, le tryptophane, la leucine, l'isoleucine, la valine, la phénylalanine, la méthionine et la thréonine, par exemple.

De préférence, la matière protéique non-traitée comprend des protéines à digestion lente, c'est à dire des protéines qui, lorsqu'elles sont ingérées par des rats de 140-200g, peuvent conduire à une disparition de la moitié de l'azote ingérée présente dans le tube digestif en plus de 80 min, par exemple. Les protéines qui coagulent dans l'estomac, comme la caséine, sont le plus souvent des protéines à digestion lente. En effet, on sait que la vitesse de digestion des protéines alimentaires est contrôlée par l'estomac, et plus précisément par la vidange gastrique (Gaudichon *et al.*, American Institute of Nutrition, Milk and Yoghurt Digestion 1970-1977, 1994). Le *coagulum* formé dans l'estomac ainsi a du mal à s'évacuer, ce qui retarde la digestion de la protéine.

Pour mettre en oeuvre le présent procédé, cette matière protéique est ensuite traitée avec la transglutaminase afin de catalyser des réactions de polymérisation, de désamidations et d'incorporation d'amines (Nielsen, Food biotechnol., <u>9</u>, 119-156, 1995). Les conditions de traitement peuvent varier selon les besoins de la présente invention. En règle générale, les protéines sont mises en suspension dans un milieu aqueux à raison de 1 à 30% en poids, on ajoute de la transglutaminase à raison de 100-100000 unités par litre, on soumet la suspension à des conditions optimales d'hydrolyse (pH7, 50°C), et on inactive l'enzyme à la chaleur et/ou par pression hydrostatique de 300-1100 MPa (à cet effet, voir EP686352 et EP748592 de S.P.N.), par exemple.

On ajoute ensuite à la matière protéique, après ou avant traitement par la transglutaminase, des polysaccharides anioniques, notamment choisis parmi les alginates, le xanthane, les gommes arabiques, les pectines, les kappa-carraghénanes, les iota-carraghénanes, les lambda-carraghénanes le carboxy-méthyl-cellulose, les dextranes sulphatés et/ou la gomme gellane. Les conditions de traitement peuvent varier selon les besoins de la présente invention. En règle générale, on ajoute entre 0,05 à 30% en poids/volume de polysaccharides anioniques, par exemple.

Les conditions de traitement de la matière protéique doivent être de préférence choisies de sorte à atteindre un niveau d'accélération de la vitesse de digestion de la matière protéique tel que, lorsque la matière protéique traitée est administrée oralement à des rats de 140-200g, elle conduit à une disparition de la moitié de l'azote ingérée présente dans le tube digestif en moins de 70 min, par exemple.

La matière protéique ainsi traitée peut être utilisée avantageusement pour la préparation d'une composition alimentaire ou pharmaceutique destinée à être administrée oralement à un mammifere afin d'induire un pic postprandial plasmatique en acides aminés, et en conséquence afin de moduler le gain protéique postprandial, et/ou de limiter les problèmes liés aux troubles de la motilité gastro-intestinale, et/ou de limiter les sensations postprandiales de nausée chez les femmes enceintes, et/ou de limiter les risques postprandiaux de régurgitation et/ou de reflux gastro-oesophagien, par exemple.

La présente utilisation n'est cependant pas limitée à une matière protéique traitée selon l'invention. En effet, d'autres traitements peuvent également induire une accélération de la vitesse de digestion d'une matière protéique. La présente utilisation vise donc aussi à utiliser toutes matières protéiques ayant été préalablement traitées de sorte à transformer les protéines à digestion lente qu'elle contenait en protéines à digestion rapide ayant conservé substantiellement au moins le même poids moléculaire initial.

Pour cela, on peut utiliser une des matières protéiques précitées, traitée cependant avec la transglutaminase, comme celles décrites dans WO97/05785 (Milupa GmBH), par exemple. Dans ce cas, le poids moléculaire des protéines ainsi traitées augmente du fait de la polymérisation des protéines.

On peut aussi utiliser uniquement des mélanges de protéines et de fibres anioniques, comme ceux décrits dans US512633, notamment en ayant recours aux polysaccharides mentionnés ci-dessus, par exemple. Dans ce cas, le poids moléculaire des protéines ainsi traitées reste identique, mais leurs propriétés physico-chimiques sont modifiées du fait de l'interaction avec les polysaccharides

De préférence, pour mettre en oeuvre la présente utilisation, on utilise une matière protéique qui contenait initialement des protéines à digestion lente, c'est à dire des protéines qui, lorsqu'elles sont ingérées par des rats de 140-200g, conduisent à une disparition de la moitié de l'azote ingérée présente dans le tube digestif en plus de 80 min.

Les matières protéiques ayant une valeur nutritive élevée sont aussi particulièrement indiquées. Ces matières peuvent être mises à profit chez les personnes ayant besoin physiologique en protéines à digestion rapide comme les patients qui sont dans une période postopératoire ou post-traumatique, les enfants ou les sportifs sortant d'un effort, ou chez les personnes ayant une répulsion physiologique à consommer des protéines à digestion lente comme les personnes âgées, les personnes souffrant d'anorexie mentale ou les femmes enceintes sujettes à des nausées, par exemple.

Les compositions alimentaires ou pharmaceutiques comprenant ces matières protéiques à digestion accélérée sont de préférence utilisées dans le cadre de régimes alimentaires, voire de traitements thérapeutiques, permettant de prévenir ou de traiter des problèmes liés à des états pathologiques ou physiologiques particuliers. Les conditions régissant ces régimes alimentaires dépendent en fait des catégories de personnes concernées. En règle générale, on considérera que la composition comprend une quantité suffisante et efficace de matières protéiques pour induire un pic plasmatique postprandial en acides aminés.

Plus particulièrement, ces compositions s'adressent aux athlètes qui cherchent à accroître leur masse corporelle, et plus spécifiquement leur masse musculaire. Ces compositions peuvent ainsi comprendre une source de protéines représentant 15% à 100% de l'énergie totale. Cette source peut être constituée essentiellement par au moins une des matières protéiques traitées décrites ci-dessus. De préférence, ces compositions comprennent de la caséine traitée, celle-ci comprenant environ 22% de valine, leucine et isoleucine, des acides aminés qui sont utilisés au niveau du muscle comme substrats énergétiques (Kasperek *et al.*, Am. J. Physiol.,_252_, E33-37, 1987) et qui peuvent permettre l'épargne des réserves glucidiques (Blomstrand *et al.,* Nutrition.,_12_, 485-490, 1996).

Les enfants qui se dépensent physiquement ont également les mêmes besoins physiologiques qu'un athlète, particulièrement après un effort intense. Les compositions alimentaires pour enfant sont de préférence formulées pour être particulièrement revitalisantes. Pour cela, elles comprennent, en plus de la source de protéines, une source de glucides rapidement assimilables, des lipides ainsi que des sels minéraux et des vitamines, par exemple.

Enfin, les patients qui sont dans une période postopératoire ou post-traumatique ont également des besoins physiologiques importants en acides aminés. Les compositions alimentaires sont de préférence formulées pour être également revitalisantes. Pour cela, elles comprennent également, en plus de la source de protéines, une source de glucides rapidement assimilables, des lipides ainsi que des sels minéraux et des vitamines, par exemple.

Les troubles de la motilité gastro-intestinale sont fréquemment associés à la vieillesse et à des états pathologiques comme l'anorexie mentale, le diabète, la présence d'un carcinome gastrique, des désordres neurologiques (la maladie de Parkinson), la dépendance aux drogues, l'alcoolisme, etc., par exemple. Le temps nécessaire à la vidange gastrique et la digestion chez ces personnes est anormalement élevé, conduisant à des symptômes d'anorexies, de nausées et de vomissements. Ces personnes peuvent ainsi présenter aussi une sensation de satiété trop élevée et prolongée après un repas de sorte qu'elles ont des difficultés à se nourrir régulièrement et suffisamment. Une composition comprenant des protéines de haute valeur nutritive, qui sont rapidement évacuées de l'estomac et qui sont rapidement digérées, participe à l'élimination de ces problèmes. Cette composition peut ainsi comprendre une source de protéines représentant 10% à 30% de l'énergie totale. Cette source de protéines peut être constituée essentiellement par au moins une des matières protéiques traitées décrites ci-dessus.

Pendant la grossesse, à cause de l'augmentation de la masse corporelle due à la conception (foetus, placenta) et l'hypertrophie de divers tissus maternels (utérus, seins, fluides extra-cellulaires), les besoins en énergie et protéines sont plus importants. Cependant, beaucoup de femmes enceintes souffrent de nausées temporaires qui les gênent pour satisfaire une diète alimentaire équilibrée. Une composition comprenant des protéines de haute valeur nutritive, qui sont rapidement évacuées de l'estomac et qui sont rapidement digérées, participe à l'élimination de ces problèmes. Cette composition peut ainsi comprendre une source de protéines représentant 10% à 30% de l'énergie totale. Cette source de protéines peut être constituée essentiellement par au moins une des matières protéiques traitées décrites ci-dessus.

Les problèmes de reflux gastro-oesophagien sont fréquemment observés chez les femmes enceintes, les nouveau-nés ou les prématurés, notamment après ingestion de lait. Les traitements actuels consistent à épaissir les aliments, et/ou à accélérer la vidange gastrique et à accroître la tonicité du sphincter gastro-oesophagien au moyen de médicaments. Les compositions selon l'invention sont particulièrement adaptées pour prévenir, voire traiter ces problèmes. Ces compositions peuvent ainsi comprendre une source de protéines représentant 10% à 40% de l'énergie totale. Cette source de protéines peut être constituée essentiellement par au moins une des matières protéiques traitées décrites ci-dessus.

Plus particulièrement, les compositions comprenant des polysaccharides anioniques présentent un triple intérêt pour traiter les problèmes de reflux. Premièrement, les matières protéiques ont une viscosité qui limite les problèmes de reflux. Deuxièmement, ces matières protéiques sont rapidement éliminées de l'estomac, ce qui limite également les problèmes de reflux. Troisièmement, ces matières protéiques sont très digestes.

Enfin, les nouveau-nés ou les patients nourris par voie entérale présentent également des problèmes de régurgitation pouvant conduire à l'obstruction des voies respiratoires. Les compositions entérales font appel à des mélanges complexes de micro- et macro-nutriments afin d'améliorer l'état nutritionnel des patients. A ce jour, on utilise généralement trois sources en acides aminés pour ces patients, à savoir des protéines intactes, des protéines hydrolysees (peptides) et des acides aminés. Les acides aminés et les peptides ont un goût désagréable, et provoquent une augmentation de l'osmolarité gastro-intestinale, un paramètre qui conduit à l'apparition de diarrhées. De plus, la valeur nutritive des acides aminés libres n'est pas comparable à celle de protéines intactes. Pour les protéines intactes, la plupart des compositions entérales utilisent la caséine. Malheureusement celle-ci coagule dans l'estomac et la vidange gastrique est ainsi prolongée. La présente invention vise à utiliser une composition ayant un bon goût, comprenant des matières protéiques non-hydrolysées qui sont digérées très rapidement. Cette composition peut ainsi comprendre une source de protéines représentant 15% à 25% de l'énergie totale. Cette source de protéines peut être constituée essentiellement par au moins une des matières protéiques traitées décrites ci-dessus.

Les compositions alimentaires ou pharmaceutiques comprennent, de préférence, une source de glucides fournissant 50 à 70% de l'énergie totale. Les glucides, notamment après un effort physique, après un traumatisme postopératoire, pendant la grossesse et chez le nouveau-née, sont des nutriments importants pour restaurer les réserves en sucre et éviter une hypoglycémie. Tous les glucides peuvent être utilisées, notamment les maltodextrines, le saccharose, le lactose et le glucose, par exemple.

Les compositions alimentaires ou pharmaceutiques peuvent comprendre une source en lipides fournissant 15 à 35% de l'énergie totale. Les huiles végétales sont recommandées, notamment provenant celles du soja, du palmier à huile, du cocotier, du tournesol, etc. Des minéraux, des vitamines, des sels, des émulsifiants ou des composés aromatiques peuvent aussi être ajoutés aux compositions, selon les besoins.

Les compositions alimentaires ou pharmaceutiques peuvent être préparées de toutes sortes de manières, les étapes de fabrication incluant généralement une dispersion des ingrédients dans de l'eau, et une pasteurisation. Les compositions peuvent être préparées sous forme de boissons ou de concentrées liquides, ou sous forme d'une poudre que l'on peut reconstituer dans de l'eau, etc., par exemple.

La présente invention est décrite plus en détail ci-après à l'aide du complément de description qui va suivre, qui se réfere à des exemples de préparation de compositions alimentaires selon l'invention. Ces exemples sont précédés d'une brève description des figures. Les pourcentages sont donnés en poids sauf indication contraire. Il va de soi, toutefois, que ces exemples sont donnés à titre d'illustration de l'objet de l'invention dont ils ne constituent en aucune manière une limitation.

- La figure 1 représente l'évolution du contenu en azote gastrique, en fonction du temps, chez des rats nourris par de la caséine native, de la caséine traitée avec la transglutaminase ou par un mélange de caséine native et de xanthane.
- La figure 2 représente l'évolution du contenu en azote gastro-intestinal (cinétique de digestion), en fonction du temps, chez des rats nourris par de la caséine native, de la caséine traitée avec la transglutaminase ou par un mélange de caséine native et de xanthane.
- La figure 3 représente l'évolution du contenu en acides aminés dans le sang, en fonction du temps, chez des rats nourris par de la caséine traitée avec la transglutaminase ou par un mélange de caséine native et de xanthane.

Exemple 1 Traitement avec la transglutaminase

On traite une suspension comprenant 10% en poids/volume de caséine micellaire avec 5 unités/ml de transglutaminase microbienne (Ajinomoto) à pH7, à 50°C et pendant 1 heure, puis on inactive l'enzyme par traitement thermique à 80°C pendant 10 min.

La suspension de caséine ainsi polymérisée ne gélifie pas, et reste stable même à pH3. Pour comparaison, la caséine micellaire native précipite à pH3 en formant des gros agrégats.

La vitesse de digestion de la caséine ainsi traitée est déterminée in vivo sur des rats mâles Sprague-Dawley (Iffa-Credo, France) pesant 140 à 200 g. Après une période d'acclimatation de 2 jours, on sépare les rats, on les fait jeûner pendant 22h, on les gave avec 5 ml d'une suspension renfermant 5% en poids de la caséine traitée, puis on endort différents lots de rats à 0 min (5 rats), 30 min (2 rats), 60 min (2 rats), 90 min (2 rats), 120 min (2 rats), 150 min (2 rats), 180 min (2 rats), 210 min (2 rats), 240 min (2 rats) ou 360 min (5 rats) après le gavage. On sacrifie les animaux, on ouvre la cavité abdominale, on prélève du sang depuis la veine porte et l'aorte dorsale, et on récupère les contenus gastrique et intestinal en lavant les différentes muqueuses avec une suspension comprenant 0,9% de chloture de sodium.

On mélange les échantillons de sang avec de l'héparine, on les centrifuge, on déprotéinise les échantillons de plasma avec une solution d'acide sulfosalycilique (3.6% conc. finale), et on congèle les échantillons jusqu'à l'analyse de leur contenu en acides aminés avec un analyseur 6300-Beckman.

On conserve au froid les contenus gastrique et intestinal, et on analyse leur teneur en azote total en mettant en oeuvre la méthode de Kjedah.

Pour comparaison, 5 rats ayant subi le même protocole sans l'étape de gavage sont utilisés pour déterminer les valeurs basales du contenu endogène en azote gastrointestinal et en acides aminés plasmatiques.

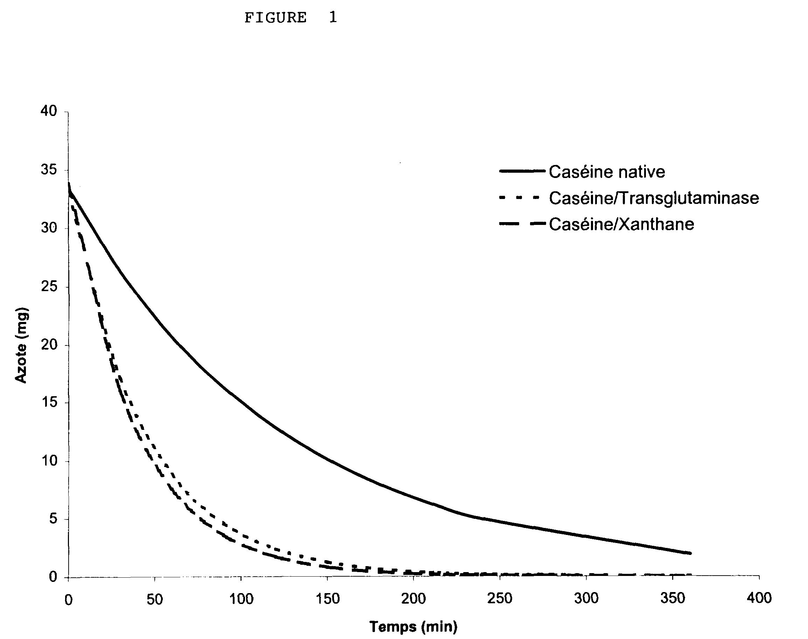

La cinétique du contenu en azote gastrique, représentée à la figure 1, montre que la moitié de l'azote ingérée disparaît de l'estomac en 35 min pour la caséine traitée, tandis qu'elle disparaît en 87 min pour la caséine native. De plus, contre toute attente, la caséine traitée reste toujours liquide dans l'estomac sans former de *coagulum*. Les paramètres de cette cinétique sont présentés dans le tableau 1 à l'exemple 2.

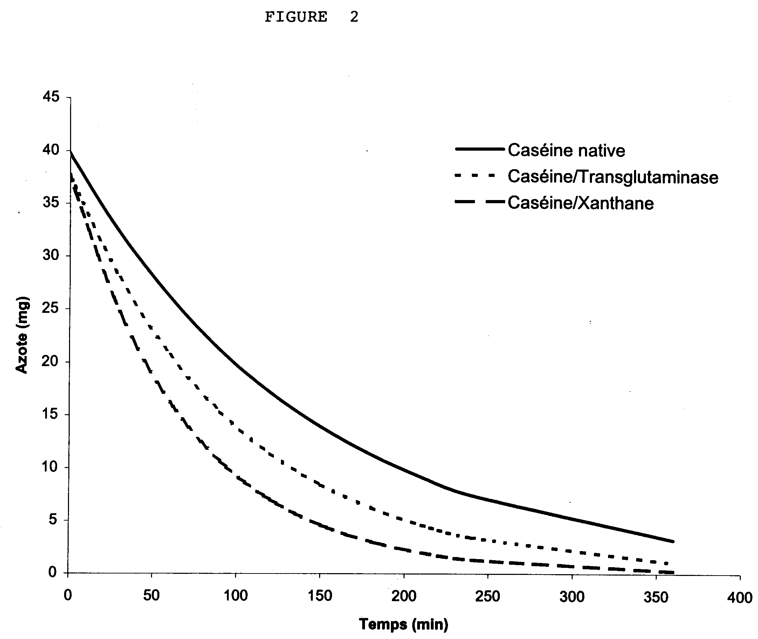

Les cinétiques de digestion sont obtenues en additionnant les valeurs du contenu en azote gastrique et en azote intestinal. Les résultats représentés à la figure 2 montrent que la moitié de l'azote ingérée disparaît du tube digestif en 69 min pour la caséine traitée, tandis qu'elle disparaît en plus de 99 min pour la caséine native. Les paramètres de cette cinétique sont présentés dans le tableau 1 à l'exemple 2.

La cinétique d'apparition des acides aminés dans le sang, représentée à la figure 3, montre l'apparition rapide d'un pic plasmatique en acides aminés pour la caséine traitée.

Exemple 2 Traitement avec des polysaccharides anioniques

On prépare plusieurs suspensions comprenant 0,1 à 10% poids/volume d'un polysaccharide anionique choisi parmi les alginates (Protanal LF20®, Pronova Biopolymers), les pectines (LM12CG®, Pomosin), le xanthane (Ketrol F®, Kelco), la gomme de guar (Guardan 178, Grinsted) et le carboxy-méthyl-cellulose (n°99-7H4XF, Aqualon), la gomme arabique (fibregum® AS IRX 29830, CNI, France), puis on ajoute 5% de caséine micellaire.

A pH3, les suspensions précipitent et forment un *coagulum* ayant une consistance mucilagineuse. L'apparence de ce coagulum est très différente de celle obtenue, à pH3, avec la caséine micellaire native qui forme des caillots à l'aspect poreux.

Les vitesses de digestion des caséines ainsi traitées sont déterminées *in vivo* sur des rats. Les résultats sont comparables à ceux présentés à l'exemple 1. A titre d'indication, les résultats obtenus plus particulièrement avec le xanthane sont exposés ci-après.

A cet effet, la cinétique du contenu en azote gastrique, représentée à la figure 1, montre que la moitié de l'azote ingérée disparaît de l'estomac en 28 min pour le mélange de caséine et de xanthane, tandis qu'elle disparaît en 87 min pour la caséine native. De plus, contre toutes attentes, la caséine traitée reste toujours liquide dans l'estomac sans former de *coagulum.* Les paramètres de cette cinétique sont présentés dans le tableau 1 ci-après.

La cinétique de digestion, représentée à la figure 2, montre que la moitié de l'azote ingérée disparaît du tube digestif en 50 min pour le mélange de caséine et de xanthane, tandis qu'elle disparaît en plus de 99 min pour la caséine native. Les paramètres de cette cinétique sont présentés dans le tableau 1 ci-après.

La cinétique d'apparition des acides aminés dans le sang, représentée à la figure 3, montre l'apparition rapide d'un pic plasmatique en acides aminés.

Tableau 1

| taux (K)[1] et t½[2] de la vidange gastrique et de la digestion pour les caséines traitée et native. | | | | |
|---|---|---|---|---|
| Caséine | Vidange gastrique | | Digestion | |
| | $K (\times 10^{-3}, min^{-1})$ | t½ (min$^{-1}$) | $K (\times 10^{-3}, min^{-1})$ | t½ (min) |
| Native | 8 | 87 | 7 | 99 |
| Caséine - Transglutaminase | 22 | 32 | 10 | 69 |
| Caséines - Xanthane | 25 | 28 | 14 | 50 |

[1] Calculé à partir de l'équation $N_t = Ae^{-Kt}$, où $N_t$= la quantité d'azote à un temps t demeurant dans l'estomac ou dans le systéme gastro-intestinal, A = le contenu en azote à t=0 et K= le taux de digestion ou de vidange gastrique.

[2] Calculé à partir de l'équation $t½ = ln2/K$, où t½= le temps requis pour que la moitié de l'azote soit éliminée de l'estomac ou soit digérée.

Exemple 3 Traitement avec la transglutaminase et des polysaccharides

On ajoute 1% de xanthane (Ketrol F [®], Kelco) à la suspension de caséine micellaire traitée avec la transglutaminase selon l'exemple 1. La vitesse de digestion de la caséine ainsi traitée est déterminée *in vivo* sur des rats comme décrit à l'exemple 1. Les résultats sont comparables à ceux obtenus dans les exemples 1 et 2.

Example 4 Composition alimentaire pour nourrissons

On prépare une composition alimentaire pour nourissons sous forme d'une poudre soluble ayant la composition définie dans le tableau 2 ci-dessous. Cette poudre est utilisée à raison de 13% dans de l'eau, ce qui correspond à une densité énergétique de l'ordre de 70 kcal/100 ml.

Pour préparer cette poudre, on purifie de l'eau par osmose inverse, on la chauffe à 70°C, on lui ajoute une source de protéines et une source de carbohydrates, on lui ajoute aussi une source de lipides dans laquelle on a préalablement dispersé les vitamines liposolubles,, on chauffe le mélange à 80°C pendant 5 min par injection de vapeur, on le refroidit à 60°C, on lui ajoute les minéraux et les vitamines hydrosolubles, on l'homogénéise en 2 étapes à 10 mPa puis à 7 mPa, on le sèche par pulvérisation sous un flux d'air chaud jusqu'à une teneur en eau de 4%, puis on le réduit en une fine poudre soluble dans l'eau.

Tableau 2

| PROTEINES | 2,3 g/100 Kcal |
|---|---|
| Caséine traitée selon l'exemple 1 | 40 % |
| Petit-lait | 60 % |
| GLUCIDES | 10 g/100 Kcal |
| Lactose | 100 % |
| LIPIDES | 5.5 g/100 Kcal |
| Graisse de lait | 70 % |
| Huile de canola | 15 % |
| Huile de mais | 14 % |
| Lécithine de soja | 1 % |
| MINERAUX | (mg/100 Kcal) |
| Sodium | 24 |
| Potassium | 100 |
| Chlorure | 67 |
| Calcium | 65 |
| Phosphore | 32 |
| Magnésium | 7 |
| Fer | 1.4 |
| Zinc | 0,7 |
| Cuivre | 0,07 |
| VITAMINES | (pour 100 Kcal) |
| Vitamine A | 300 IU |
| Vitamine D | 65 IU |
| Vitamine E | 1,3 IU |
| Vitamine K | 8,2 µg |
| Vitamine C | 8,1 mg |
| Thiamine | 60 µg |
| Riboflavine | 130 µg |
| Niacine | 760 µg |
| Vitamine B6 | 70 µg |
| Acide folique | 10 µg |
| Acide Pantothénique | 460 µg |
| Vitamine B12 | 0,3 µg |
| Biotine | 2,5 µg |
| Choline | 8 mg |
| Inositol | 5 mg |

<u>Exemple 5</u> Supplément alimentaire pour sportifs

On prépare un supplément alimentaire liquide destiné aux sportifs et plus particulièrement aux personnes qui viennent d'effectuer une activité physique. Le processus de fabrication est similaire à celui décrit dans l'exemple 4. Ce produit liquide nutritionnel présente une densité énergétique de 60 Kcal/100 ml. La composition de cette formule est détaillée dans le tableau 3 ci-dessous.

Tableau 3

| PROTEINS | 5 g/100 Kcal |
|---|---|
| Caséine traitée selon l'exemple 1 | 100% |
| GLUCIDES | 10 g/100 Kcal |
| Maltodextrines | 64% |
| Saccharose | 36% |
| MINERAUX | mg/100 ml |
| Sodium | 20 |
| Potassium | 45 |
| Calcium | 15 |
| Magnésium | 7 |
| Phosphore | 17 |
| Fer | 0.5 |
| Iode | 0,01 |
| Sélénium | 0,02 |
| VITAMINES | mg/100 ml |
| C | 30 |
| B1 | 0,25 |
| B2 | 0,33 |
| B5 | 1,4 |
| B6 | 0,35 |
| B12 | 0,8 |
| Biotine | 28 |
| D3 | 0,8 |
| K | 8 |
| PP | 3,5 |

<u>Example 6</u> Composition entérale

On prépare une composition entérale liquide ayant les ingrédients définis au tableau 4 ci-dessous, de la même manière qu'à l'exemple 4 à la différence près qu'on homogénéise le mélange à 150°C par injection de vapeur, on le refroidit à 75°C et on le conditionne aseptiquement dans des récipients. Cette composition présente une densité énergétique de 100 Kcal/100 ml.

Tableau 4

| PROTEINES | 6,5 g/100 ml |
|---|---|
| Caseine traitée selon l'exemple 2 (xanthane: caséine, rapport 5:1) | 100 % |
| GLUCIDES | 11,3 g/100 ml |
| Solides d'un sirop de maïs | 66 % |
| Saccharose | 34% |
| LIPIDES | 3,4 g/100 ml |
| Huile de noix de coco | 50 % |
| Huile de canola | 30 % |
| Huile maïs | 14 % |
| Lécithine de soja | 6 % |
| MINERAUX | (mg/100 ml) |
| Calcium | 80 |
| Potassium | 150 |
| Phosphore | 70 |
| Chlorure | 130 |
| Sodium | 87,6 |
| Zinc | 1,4 |
| Magnésium | 40 |
| Fer | 1,2 |
| Cuivre | 0,2 |
| Manganèse | 0,3 |
| Sélénium | 0,1 |
| Chrome | 0,004 |
| Molybdène | 0,012 |
| Iode | 0,01 |
| VITAMINES | (pour 100 ml) |
| VitamineA | 400 IU |
| $\beta$-carotène | 0,2 mg |
| Vitamine D | 30 IU |
| Vitamine E | 4 IU |
| Vitamine C | 15 mg |
| Vitamine K | 8 µg |
| Thiamine | 0,2 mg |
| Riboflavine | 0,25 mg |
| Niacine | 2,4 mg |
| Pyridoxine | 0,4 mg |
| Acide folique | 50 µg |

Tableau 4 (continued)

| Acide Pantothénique | 1,4 mg |
|---|---|
| Vitamine B12 | 0,8 µg |
| Biotine | 40 µg |
| Choline | 45 mg |

Example 7 Composition nutritionelle pour les enfants

On prépare une composition nutritionelle liquide pour des enfants et/ou des adolescents sous forme d'un lait aromatisé au chocolat. Cette composition présente une densité énergétique de 75 Kcal/100 ml, et ses constituents sont précisés au tableau 5 ci-dessous. Elle est préparée de la même manière qu'à l'exemple 4 à la différence près qu'on homogénéise le mélange à 150°C par injection de vapeur, on le refroidit à 75°C et on le conditionne aseptiquement dans des récipients Tetra-Brick®.

Tableau 5

| PROTEINE | 4 g/100 Kcal |
|---|---|
| Caséine traitée selon l'exemple 1 | 100 % |
| GLUCIDES | 15 g/100 Kcal |
| Solides d'un sirop de maïs | 62 % |
| Saccharose | 38 % |
| LIPIDES | 2,5 g/100 Kcal |
| Graisses de lait | 40 % |
| Huile de maïs | 42 % |
| Beurre de cacao | 10 % |
| Lécithine de soja | 8 % |
| MINERAUX | (mg/100 Kcal) |
| Calcium | 50 |
| Phosphore | 30 |
| Zinc | 0,3 |
| Magnésium | 4 |
| Fer | 0,5 |
| Selénium | 0,02 |
| Zinc | 0,7 |
| Iode | 0,01 |
| VITAMINES | (pour 100 Kcal) |
| Vitamine A | 250 IU |
| Vitamine D | 50 IU |
| Vitamine E | 1 IU |
| Vitamine C | 8 mg |
| Thiamine | 60 µg |
| Riboflavine | 130 µg |
| Niacine | 760 µg |
| Vitamine B6 | 70 µg |
| Acide folique | 10 µg |
| Acide Pantothénique | 460 µg |
| Vitamine B12 | 0,8 µg |

Example 8 Substitut de lait pour les femmes enceintes

On prépare une composition en poudre destinée à substituer le lait de vache pour la nutrition des femmes enceintes souffrant de problèmes de nausées et/ou de vomissements. Cette poudre peut être reconstituée dans de l'eau à raison de 10,7 %. La densité énergétique de la composition reconstituée est de l'ordre de 50 kcal/100 ml. Les ingrédients de la composition sont précisés au tableau 6 ci-dessous. Cette composition est préparée de la même manière qu'à l'exemple 4.

Tableau 6

| PROTEINES | 4.0 g/100 Kcal |
|---|---|
| Caséine traitée de l'exemple 3 (xanthane: caséine, 1:5) | 40 % |
| Petit-lait | 60 % |
| GLUCIDES | 14,8 g/100 Kcal |
| Maltodextrin | 55 % |
| Saccharose | 45 % |
| LIPIDES | 2,7 g/100 Kcal |
| Graisse de lait | 20 % |
| Huile de maïs | 74 % |
| Lécithine de soja | 6 % |
| MINERAUX | (mg/100 Kcal) |
| Calcium | 280 |
| Potassium | 60 |
| Phosphore | 140 |
| Chlorure | 60 |
| Sodium | 30 |
| Zinc | 6 |
| Magnésium | 23 |
| Fer | 5 |
| Iode | 0,05 |
| VITAMINES | (pour 100 Kcal) |
| Vitamine A | 250 IU |
| Vitamine D | 120 IU |
| Vitamine E | 4,7 IU |
| Vitamine C | 35 mg |
| Thiamine | 0,4 mg |
| Riboflavine | 0,4 mg |
| Niacine | 5 mg |
| Pyridoxine | 0,8 mg |
| Acide folique | 150 µg |
| Acide Pantothénique | 1,2 mg |

Example 9 Composition nutritionelle pour personnes âgées

On prépare une composition en poudre destinée à substituer le lait de vache pour la nutrition des personnes âgées souffrant de troubles de la motilité gastrointestinale. Cette poudre peut être reconstituée dans de l'eau à raison de 10,7 %. La densité énergétique de la composition reconstituée est de l'ordre de 50 kcal/100 ml. Les ingrédients de la composition sont présentés au tableau 7 ci-dessous. Cette composition est préparée de la même manière qu'à l'exemple 4.

Tableau 7

| PROTEINES | 3,0 g/100 Kcal |
|---|---|
| Caséine traitée selon le procédé de l'exemple 3 avec un kappa-carraghénane (kappa-carraghénane:caséine, 1:5) | 45 % |
| Petit-lait | 55 % |
| GLUCIDES | 15,3 g/100 Kcal |
| Maltodextrin | 70 % |
| Saccharose | 30 % |
| LIPIDES | 3,0 g/100 Kcal |
| Graisses de lait | 20 % |
| Huile de maïs | 74 % |
| Lécithine de soja | 6 % |
| MINERAUX | (mg/100 Kcal) |
| Calcium | 200 |
| Phosphore | 120 |
| Chlorure | 50 |
| Sodium | 20 |
| Zinc | 4 |
| Magnésium | 20 |
| Fer | 4 |
| Iode | 0,05 |
| VITAMINES | (pour 100 Kcal) |
| Vitamine A | 150 IU |
| Vitamine D | 120 IU |
| Vitamine E | 3 IU |
| Vitamine C | 40 mg |
| Thiamine | 0,4 mg |
| Riboflavine | 0,3 mg |
| Niacine | 5 mg |
| Pyridoxine | 0,75 mg |
| Acide folique | 75 $\mu$g |
| Acide Pantothénique | 1 mg |

**Revendications**

1. Procédé pour accélérer la vitesse de digestion d'une matière protéique, dans lequel on traite une matière protéique avec la transglutaminase et on la mélange avec des polysaccharides anioniques.

2. Procédé selon la revendication 1 dans lequel, les polysaccharides anioniques sont choisis parmi les alginates, le xanthane, les gommes arabiques, les pectines, les kappa-carraghénanes, les iota-carraghénanes, les lambdacarraghénanes le carboxy-méthyl-cellulose, les dextranes sulphatés et/ou la gomme gellane.

3. Procédé selon la revendication 1 dans lequel, la matière protéique comprend des protéines de lait.

4. Procédé selon la revendication 1 dans lequel, la matière protéique comprend des protéines à digestion lente, c'est à dire des protéines qui, lorsqu'elles sont ingérées par des rats de 140-200g, conduisent à une disparition de la moitié de l'azote ingérée présente dans le tube digestif en plus de 80 min.

5. Procédé selon la revendication 1 dans lequel, le traitement de la matière protéique par la transglutaminase et l'adjonction de polysaccharides anioniques sont tels que la matière protéique ainsi traitée, lorsqu'elle est ingérée par des rats de 140-200g, conduit à une disparition de la moitié de l'azote ingérée présente dans le tube digestif en moins de 70 min.

6. Utilisation d'une matière protéique à digestion rapide, c'est à dire une matière qui, lorsqu'elle est ingérée par des rats de 140-200g, conduit à une disparition de la moitié de l'azote ingérée présente dans le tube digestif en moins de 70 min,
   pour la préparation d'une composition alimentaire ou pharmaceutique destinée à être administrée oralement à un mammifere afin de moduler le gain protéique postprandial,
   ladite matière protéique ayant été préalablement traitée de sorte à transformer les protéines à digestion lente qu'elle contenait en protéines à digestion rapide ayant conservé substantiellement au moins le même poids moléculaire initial.

7. Utilisation selon la revendication 6, pour la préparation d'une composition alimentaire destinée à être consommée par des patients qui sont dans une période postopératoire ou post-traumatique, par des enfants ou par des sportifs, de sorte à assurer le maintien, la restauration ou le développement de leur masse protéique.

8. Utilisation selon la revendication 7, caractérisée en ce que la composition est composition revitalisante destinée à assurer le maintien, la restauration ou le développement de leur masse musculaire.

9. Utilisation d'une matière protéique à digestion rapide, c'est à dire une matière qui, lorsqu'elle est ingérée par des rats de 140-200g, conduit à une disparition de la moitié de l'azote ingérée présente dans le tube digestif en moins de 70 min,
   pour la préparation d'une composition alimentaire ou pharmaceutique destinée à être administrée oralement à un mammifere afin de réduire les problèmes liés aux troubles de la motilité gastro-intestinale,
   ladite matière protéique ayant été préalablement traitée de sorte à transformer les protéines à digestion lente qu'elle contenait en protéines à digestion rapide ayant conservé substantiellement au moins le même poids moléculaire initial.

10. Utilisation selon la revendication 9, caractérisée en ce que la composition est destinée aux personnes présentant une sensation de satiété trop élevée et prolongée après un repas de sorte qu'elles ont des difficultés à se nourrir régulièrement et suffisamment.

11. Utilisation selon la revendication 9, caractérisée en ce que l'on traite les personnes âgées ou les personnes présentant un état pathologique comme l'anorexie mentale, le diabète, la présence d'un carcinome gastrique, des désordres neurologiques, la dépendance aux drogues ou l'alcoolisme.

12. Utilisation d'une matière protéique à digestion rapide, c'est à dire une matière qui, lorsqu'elle est ingérée par des rats de 140-200g, conduit à une disparition de la moitié de l'azote ingérée présente dans le tube digestif en moins de 70 min,
   pour la préparation d'une composition alimentaire ou pharmaceutique destinée à être administrée oralement afin de limiter les sensations de nausée chez les femmes enceintes,
   ladite matière protéique ayant été préalablement traitée de sorte à transformer les protéines à digestion lente qu'elle contenait en protéines à digestion rapide ayant conservé substantiellement au moins le même poids moléculaire initial.

13. Utilisation d'une matière protéique à digestion rapide, c'est à dire une matière qui, lorsqu'elle est ingérée par des rats de 140-200g, conduit à une disparition de la moitié de l'azote ingérée présente dans le tube digestif en moins de 70 min,
   pour la préparation d'une composition alimentaire ou pharmaceutique destinée à être administrée oralement à un mammifere afin limiter les risques de régurgitation et/ou de reflux gastro-oesophagien,

ladite matière protéique ayant été préalablement traitée de sorte à transformer les protéines à digestion lente qu'elle contenait en protéines à digestion rapide ayant conservé substantiellement au moins le même poids moléculaire initial.

14. Utilisation selon la revendication 13, caractérisée en ce que la composition est destinée en particulier à la nutrition infantile, la nutrition des femmes enceintes ou la nutrition entérale.

15. Utilisation selon l'une des revendication 6 à 14, caractérisée en ce que les protéines à digestion lente contenues initialement dans la matière protéique sont des protéines qui, lorsqu'elles sont ingérées par des rats de 140-200g, conduit à une disparition de la moitié de l'azote ingérée présente dans le tube digestif en plus de 80 min.

16. Utilisation selon l'une des revendication 6 à 14, caractérisée en ce que la matière protéique à digestion rapide est choisie parmi les matières protéiques traitées par la transglutaminase, les matières protéiques mélangées avec des polysaccharides anioniques et/ou les matières protéiques obtenues selon le procédé decrit aux revendications 1 à 5.

17. Composition alimentaire ou pharmaceutique comprenant des polysaccharides anioniques et une matière protéique traitée par la transglutaminase.

FIGURE 1

FIGURE 2

Azote (mg)

Temps (min)

——— Caséine native
– · · – Caséine/Transglutaminase
— — Caséine/Xanthane

FIGURE 3

**EP 0 920 813 A1**

| Office européen des brevets | **RAPPORT DE RECHERCHE EUROPEENNE** | Numéro de la demande<br>EP 97 20 3840 |
|---|---|---|

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| A | WO 93 22930 A (NOVO NORDISK) 25 novembre 1993<br>* page 3, ligne 27-31; revendications *<br>--- | 1,3,17 | A23L1/305<br>A23L1/05 |
| A | EP 0 725 145 A (DRUG DELIVERY SYSTEM INSTITUTE) 7 août 1996<br>* page 2, ligne 12-15; revendication 1 *<br>--- | 1,17 | |
| A | EP 0 492 406 A (RÖHM) 1 juillet 1992<br>* revendications *<br>--- | 1,17 | |
| D,A | WO 97 05785 A (MILUPA) 20 février 1997<br>* revendications *<br>--- | 1,17 | |
| D,A | US 5 126 332 A (A.OHTA ET AL.) 30 juin 1992<br>* revendications *<br>----- | 1-3,17 | |

**DOMAINES TECHNIQUES RECHERCHES (Int.Cl.6)**

A23L

Le présent rapport a été établi pour toutes les revendications

| Lieu de la recherche | Date d'achèvement de la recherche | Examinateur |
|---|---|---|
| LA HAYE | 4 juin 1998 | Van Moer, A |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)